# EUROPEAN PATENT APPLICATION

(11) **EP 1 300 683 A1**
(43) Date of publication of application: **09.04.2003**
(21) Application number: 01949935.9
(22) Date of filing: 11.07.2001
(51) Int. Cl.: G01N 33/53, G01N 33/92

(54) **MEANS OF EXAMINING NEPHROPATHY**

(30) Priority: 13.07.2000 JP 2000212431; 08.02.2001 JP 2001031746
(71) Applicant: International Reagents Corporation, Kobe-shi Hyogo 651-2271 (JP)
(72) Inventor: HOTTA, Osamu, Sendai-shi, Miyagi 983-0823 (JP); SHIRAHASE, Yasushi, c/o Res. & Dev. Center Int., Kobe-shi, Hyogo 651-0083 (JP); HIURA, Hisahide, c/o Res. & Dev. Center Int., Kobe-shi, Hyogo 651-0083 (JP)
(74) Representative: Forstmeyer, Dietmar, Dr. rer. nat., Dipl.-Chem.
(86) International application number: JP0106006
(87) International publication number: WO02006832

(57) **Abstract**

A convenient means of examining nephropathy is provided. Paying attention to fat granules Contained in urinary lipomicrons and oval fat which are observed in association with nephropathy, it is found out that the contents of lipoproteins or analogs thereof or constituents of the same contained in these substances significantly indicate relevance to nephropathy, thereby completing the above-described examination means.

## Description

### Technical Field

The present invention relates to a examination of a nephropathy, more specifically relates to the examination by measurement of a lipoprotein or analogs thereof contained in urine or a constituent contained therein.

### Background Art

Urinary sediment contains various constituents such as constituents derived from a kidney, constituents added from a urinary passage, and also crystalline constituents sedimented in urine. Analyzing a species and an amount of sediment is very important for diagnosis of renal and urinary passage diseases and determination of a degree thereof. Analyses thereof are commonly conducted by a microscopic observation applying a staining method. On the other hand, it has been pointed out that examination of a surface antigen of a macrophage contained in urine allows diagnosing a lesion of IgA nephritis (Rinsyou Kensa 42: 588 - 590, 1998), and detection of a urinary fat drop and an oval fat body (hereafter, may be simply called a fat body) by urinary sediment fat staining allows an examination of nephropathy (Kensa to Gizyutu 26: 441 - 446, 1998).

In addition, a giant macrophage appearing in urine shows negative surface antigen CD14 and positive 25F9 of a leukocyte and differs largely from a normal macrophage showing positive surface antigen CD14 and negative 25F9. Further, it was shown that the fat drop and the oval fat body belong to the giant macrophage and mentioned that measuring them is useful for examining nephropathy (Rinsyou Kensa 47, Special edition for Annual Meeting: 73, 1999).

These measurements were all conducted by microscopic observation of a stained specimen, microscopic observation through fluorescence labeled immunological staining using a monoclonal antibody or flow cytometry. However, in these methods, the examination was carried out by microscopic observation or flow cytometry for every specimen and thus, operations are complicated to make measurement of a large number of samples difficult.

### Disclosure of the Invention

An object of the present invention is to provide convenient means for examining nephropathy.

The present inventors measured a very small amount of cholesterol (mCHO) on the basis of the idea that the giant macrophage as described above might be measured by using mCHO and then, found the specimen of which mCHO showed a high value even in a low level of the giant macrophage. In a patient showing an appearance of lipoprotein, the disease rapidly progressed and showed transfer to renal failure with a high probability. Then, as a result of our intensive studies, we found that a lipoprotein or analogs thereof or the amount of constituents or particularly of an apoprotein contained therein significantly suggests a relationship with nephropathy resulting in completion of the means, according to the invention, for examining nephropathy.

Consequently, the invention comprises:
1. Method for examining nephropathy, characterized by measuring a lipoprotein or an analog thereof contained in urine, or a constituent contained the lipoprotein or the analog;
2. The method for examining nephropathy according to claim 1, characterize in that the lipoprotein or the analog thereof is a substance containing at least one protein selected from the group consisting of a protein reactable with an anti-human apoA-I antibody and a protein reactable with an anti-human apoA-II antibody;
3. The method for examining nephropathy according to claim 1 or 2, characterized in that the lipoprotein or the analog thereof is a high density lipoprotein or an analog thereof;
4. The method for examining nephropathy according to any of claims 1 to 3, characterized by measuring an apoprotein as the constituent contained in the lipoprotein or the analog thereof;
5. The method for examining nephropathy according to any of claims 1 to 4, characterized by measuring at least one protein selected from the group consisting of a protein reactable with an anti-human apoA-I antibody and a protein reactable with an anti-human apoA-II antibody as the constituent contained in the lipoprotein or the analog thereof;
6. The method for examining nephropathy according to any of claims 1 to 5, characterized by further measuring a surface antigen of a urine leukocyte;
7. The method for examining nephropathy according to any of claims 1 to 6, characterized by further measuring a lipid constituent;
8. The method for examining nephropathy according to claim 7, characterized in that the lipid constituent is cholesterol;
9. A reagent for a examination, comprising a kit or a single product of a reagent necessary for the examination according to any of foregoing paragraphs 1 to 8;

### Brief Description of the Drawings

Fig. 1 shows a relationship between a urine lipid-related marker (an oil red-positive particle) and a disease. (Example 3)
Fig. 2 shows the relationship between a urine lipid-related marker (free cholesterol) and a disease. (Example 3)
Fig. 3 shows the relationship between a urine lipid-related marker (apoA-I) and a disease. (Example 3)
Fig. 4 shows the relationship between a urine lipid-related marker (phospholipid) and a disease. (Example 3)
Fig. 5 shows the relationship between a urine lipid-related marker (total cholesterol) and a disease. (Example 3)
Fig. 6 shows a result of an HPLC analysis of a cholesterol sample. (Example 5)
Fig. 7 shows a result of an HPLC analysis of concentrated normal urine. (Example 5)
Fig. 8 shows a result of an HPLC analysis of urine of a renal disease patient. (Example 5)
Fig. 9 shows measured values of urine mCHO and serum creatinine of the renal disease patient before treatment and after treatment. (Example 6)
Fig. 10 shows measured values of urine mCHO and urine N-acetyl β-D-glucosaminidase of the renal disease patient before treatment and after treatment. (Example 6)
Fig. 11 shows measured values of urine mCHO and urine protein of the renal disease patient before treatment and after treatment. (Example 6)
Fig. 12 shows measured values of urine mCHO and urine albumin of the renal disease patient before treatment and after treatment. (Example 6)
Fig. 13 shows measured values of urine mCHO and urine apoA-I of the renal disease patient before treatment and after treatment. (Example 6)

### Description of the symbols

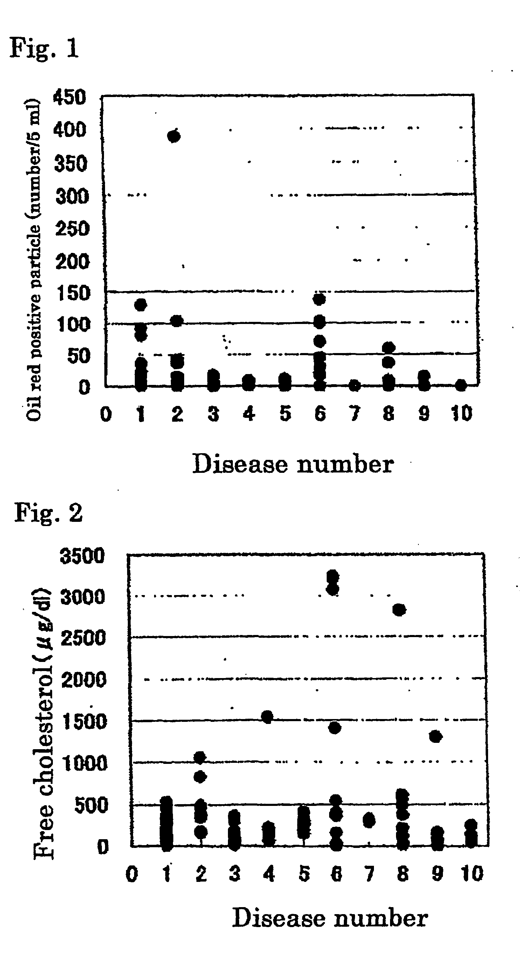

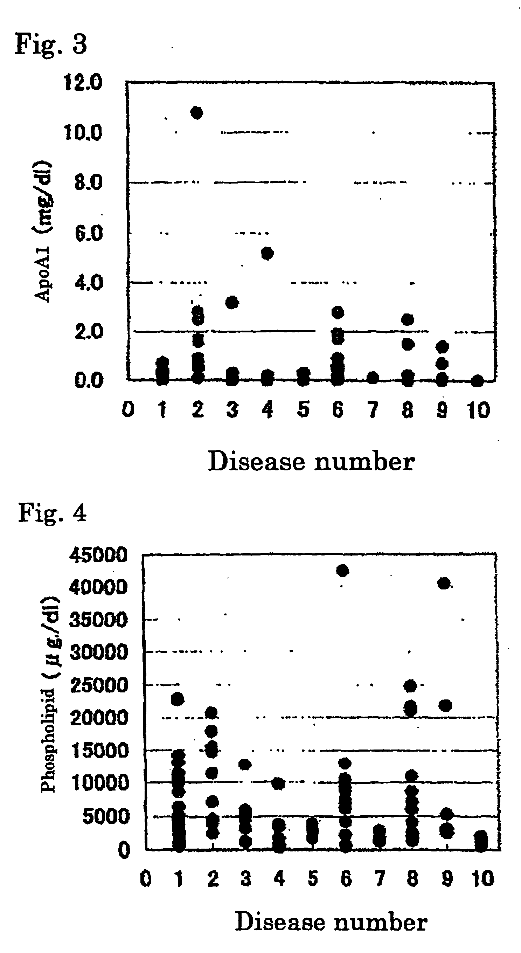

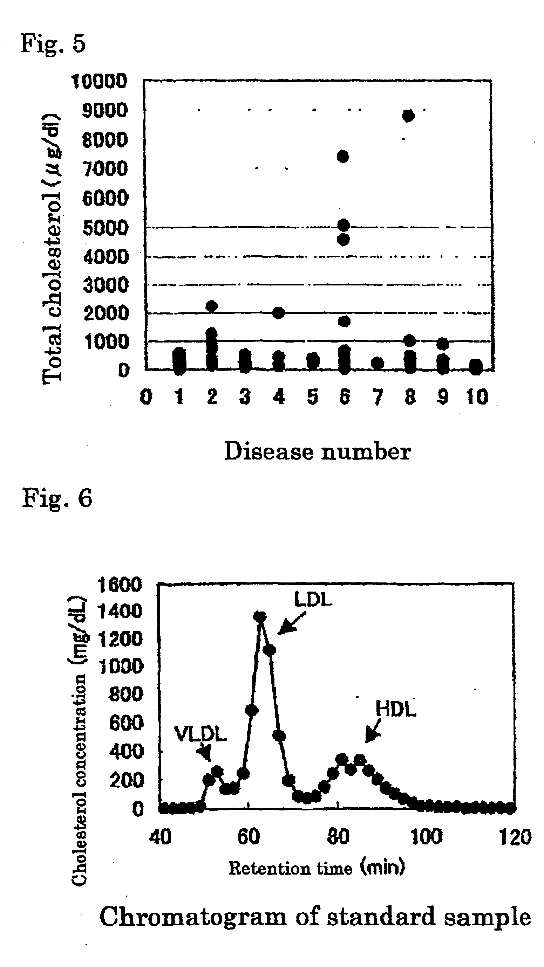

### (For Figs 1 to 5)

1: IgA nephropathy
2: diabetic nephropathy
3: glomerulosclerosis
4: membranous nephropathy
5: membranoproliferative glomerulonephritis disease
6: rapidly progressive nephropathy
7: minimal change nephrosis
8: focal glomerulosclerosis
9: chronic renal failure
10: Normal

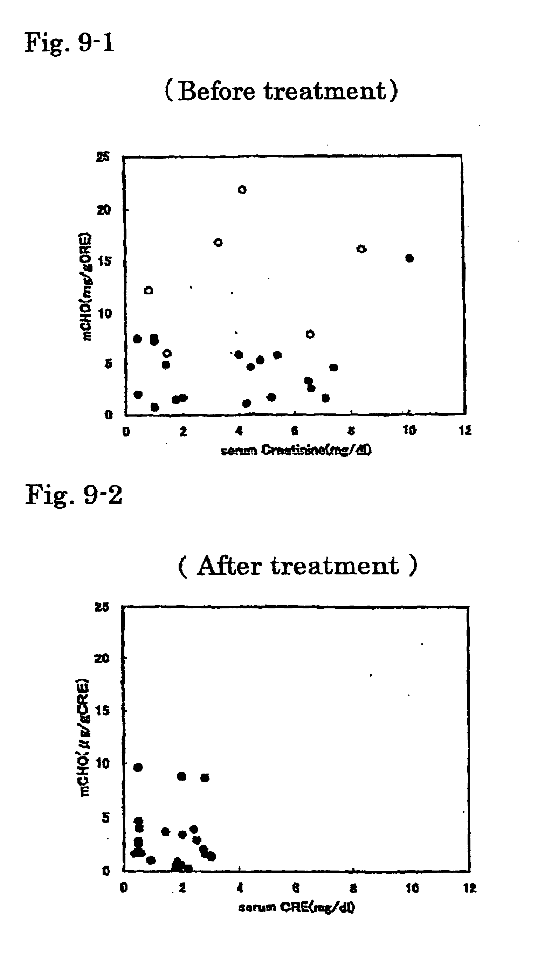

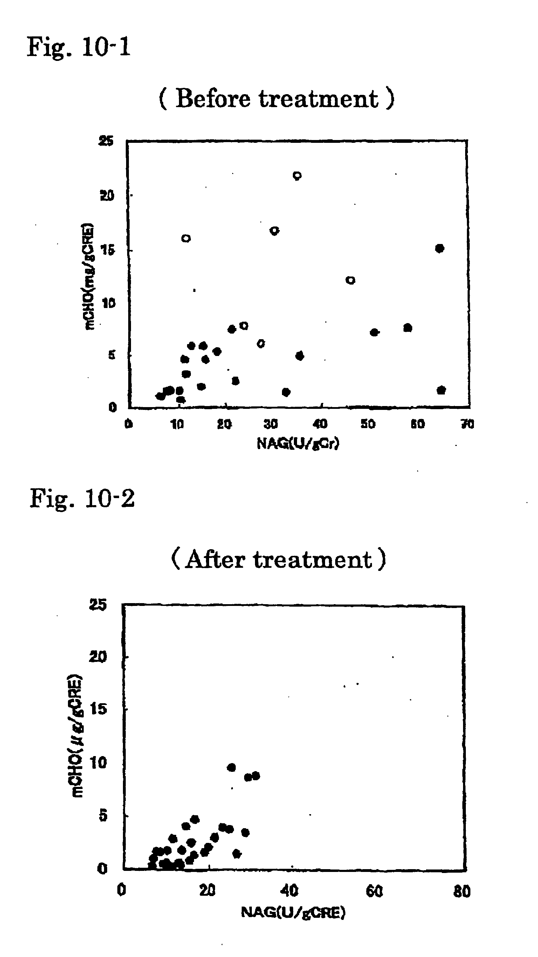

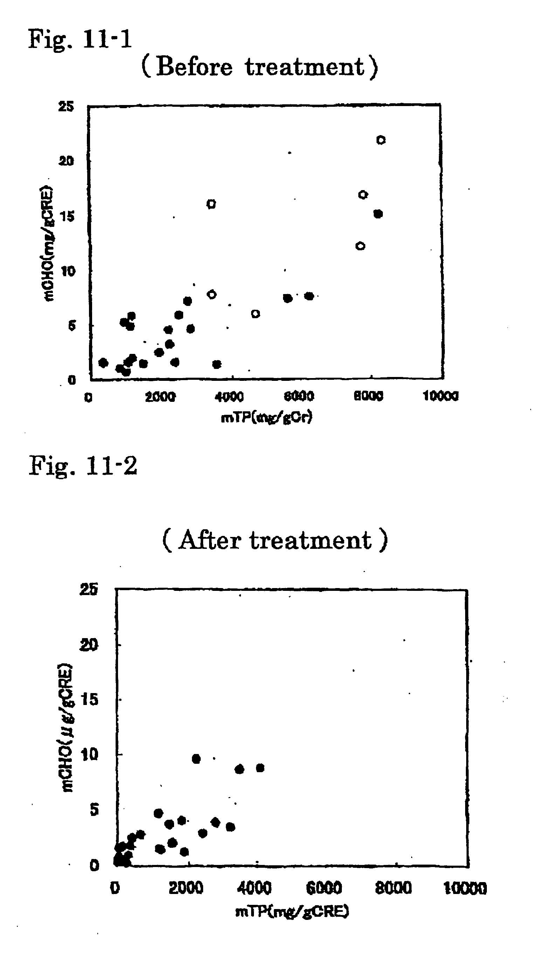

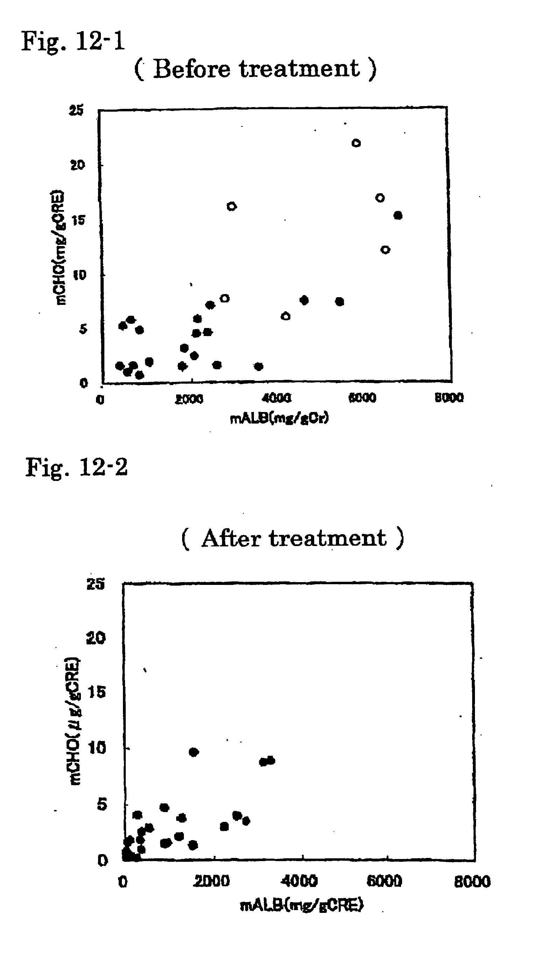

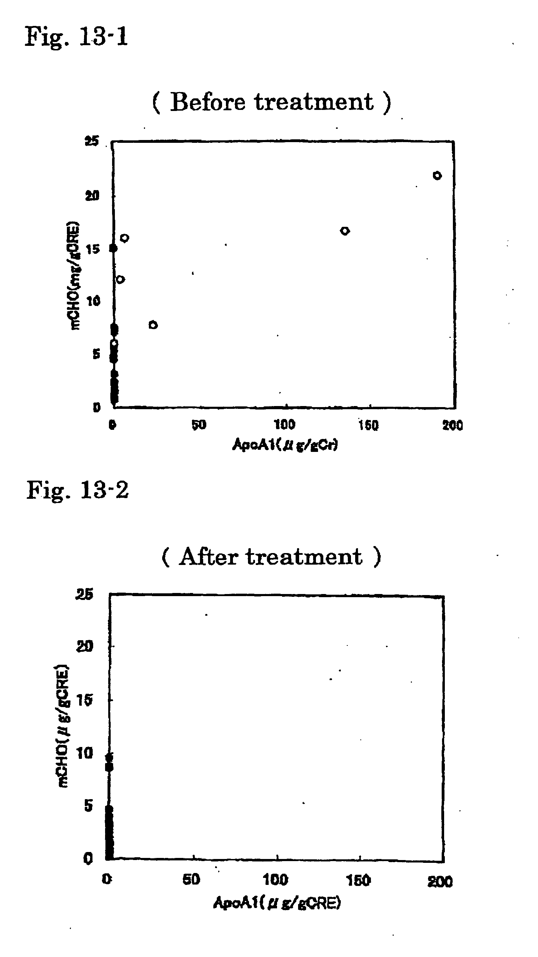

### (For Figs 9 to 13)

●: value measured for an apoA-I negative patient
○: value measured for an apoA-I positive patient

### Best Mode for Carrying Out the Invention

The invention is a means for examining a nephropathy, characterized by measuring a lipoprotein or analogs thereof contained in urine or a constituent contained therein. Herein, the means is defined as a method and/or a medium for achieving an object.

In other words, the examination means for a nephropathy according to the invention is achieved by measuring a lipoprotein or an analog thereof contained in urine, or a constituent contained therein. In addition to these substances, the invention provides also examination means for a nephropathy characterized by measuring a lipid constituent contained in urine.

Among the lipid constituents contained in urine, a fat droplet, a fat body, and/or those included in the fat drop, and a lipoprotein like a high density lipoprotein (HDL-like) is a major object for measurement. Particularly in renal diseases, we found newly that a HDL-like lipoprotein appears in urine. In serum, lipids are present in a form of a lipoprotein, however, in urine, frequently in particles (fat droplet, fat body, and/or fat drop) in a degree visible by a microscope. For these insoluble fat masses, the invention makes quantification of an amount of a fat constituent possible by an enzyme reaction.

### (Objective lipoprotein or analogs thereof, or constituents contained therein)

In the invention, lipoprotein or analogs thereof contained in urine or constituents contained therein, which are major objects of the measurement, are as follows. A complex containing a lipid and a protein in a specific proportion is called lipoprotein, which includes a chylomicron, VLDL, IDL, LDL, HDL, and the like, however, is not restricted to these compounds. A lipid constituent is almost same as that described later (objective lipid constituent,) and, includes mainly cholesterols, triacyl glycerols, phospholipids, free fatty acids, and the like. A part of these lipids shows very high hydrophobicity and thus, forms a special structure called a lipoprotein and arranges a special protein called apoprotein on a surface thereof. The apoprotein contributes importantly to structural determination of the lipoprotein and also contributes importantly to a lipoprotein metabolism.

As major apoproteins of various lipoproteins, chylomicron includes A-1, B, C-I, II, and III, VLDL includes B, C-I, II, III, and E, IDL includes B and E, LDL includes B, and HDL2 and HDL3 include A-I and II, which are known as structural constituents.

The analog of lipoprotein in the invention includes those having difference electric charges, those modified by a sugar or other substances, those having different molecular weights, and those having similar amino acid sequences, for example.

In the invention, the protein reactable with the anti-human apoA-I antibody or the protein reactable with the anti-human apoA-II antibody includes a human apoA-I protein or a human apoA-II protein itself and also a protein reactable with the antibody as described above through having a structure common to these proteins.

### (Lipid constituent)

The urine lipid constituent as the object for measurement in the invention is one or more species of lipid constituents selected from, for example, a neutral fat and a derivative thereof, a phospholipid and the derivative thereof, a lipid peroxide, sterols, fatty acids, fatty acid salts, fatty acid esters, aliphatic alcohols, aliphatic aldehydes, lipoids, sphingolipids, prostaglandins, carotinoids, and the like and a concentration or content thereof is measured by working an enzyme acting to these lipid constituents. Particularly important objects among lipid constituents are sterols and/or phospholipids.

### (Treatment of a sample for measurement)

The urine sample may be urine itself and may be a urine sediment fraction yielded by centrifugation of urine or a supernatant fraction thereof. A method for measuring the lipid constituent according to the invention not only makes possible a direct measurement of the urine sample without any treatment, but also makes possible the measurement of the lipid constituent of a faction yielded by fractionation of the urine sediment produced by centrifugation of the urine sample.

### (Method for measurement)

Measurement of lipoprotein or analogs thereof contained in urine or the constituent contained therein is sufficiently conducted by a measuring method detectable of these substances and not specially restricted. For the measurement, a surfactant may be used or not used. For example, for measurement of lipoprotein or analogs thereof, it is preferable for measurement to use no surfactant. In the case where the constituent contained. therein is measured, the measurement using the surfactant is preferable.

### (Method for measuring lipoprotein)

Lipoprotein may be measured by fractionation of lipoprotein by, for example, HPLC, ultracentrifugation, and agglutination using a polyethylene glycol to measure the lipoprotein fractionated by weight method.

### (Method for measuring apoprotein)

On the other hand, for measurement of apoprotein, measurement by antigen-antibody reaction may be applied; for example, for apoA-I protein, immunonephelometry, particle agglutination immunity measurement method, enzyme immunity measurement method, and the like, which are publicly known, may be applied using the anti-apoA-I protein antibody. Other apoprotein is exemplified by apoA-II, apoB, apoC, apoE, apoLP (a), and the like.

### (Addition of surfactant)

The surfactant is not specially restricted, if it is sufficient to be a substance having actions such as solubilizing or dispersing and homogenizing an object, which is contained in urine, of measurement to homogenize the object for measurement in order to allow choosing any part of urine, and/or making a state possible for quantification by the enzyme and the antibody. Preferable substances are those having the action solubilizing fat droplet, fat body, fat drop, and lipoprotein and making possible homogenization of the lipid constituents, proteins, insoluble lipids, and the like, which are contained therein, through solubilizing, dispersing, releasing. In addition, ultracentrifugation may be applied independently or combined with surfactant treatment.

In specific example of the invention, triton X-100 has been exemplified as a nonionic surfactant and is not specially restricted thereto. Those skilled in the art can perform a comparison of effects by repeating simply experiments on the basis of a result disclosed in the invention and can select the surfactant. Other than the nonionic surfactant, cationic surfactant, anionic surfactant, ampholytic surfactant, or a glycoside may be properly used. In addition, a combined use of two or more species of surfactants allows achieving a homogenizing effect through ensuring solubilization and dispersion.

An amount of the surfactant to be added ranges generally from 0.1 to 5 w/v and in consideration of a degree of homogenization through solubilization and dispersion, may be selected through increasing and decreasing the amount properly. A temperature and time as a treatment condition may be determined by simple experimental repetition. On the other hand, preferably, it is possible to add previously the surfactant to urine as a pretreatment to solubilize or disperse the object for homogenizing treatment. In this event, it is sufficient to treat at 10 to 40 deg. C temperature for several seconds to several minutes. Of course, this surfactant treatment may be simultaneously conducted with the enzyme reaction treatment. Concerning the treatment, stepwise treatments may be properly applied and more preferably, an automated condition or a unified treatment is applied.

### (Quantification of lipid constituent by using enzyme)

The lipid constituent contained in urine may be measured by a measuring method detectable of the urine lipid constituent. The lipid constituent contained in urine, in comparison with that contained in serum, ranges only about 1/1000 to 1/100 and is difficult to be measured by using a reagent for measurement of a serum lipid.

As described above, the measurement of the urinary lipid constituent homogenized by solubilization or dispersion by adding the surfactant or the like is conducted by employing the method for quantifying the lipid using the enzyme. The method being a publicly known method is not specially restricted. For example, HPLC method, gas chromatographic method, electrophoretic method, chemical method, and the method using the enzyme are exemplified. Among these methods, the method using the enzyme is preferably used in view of operability, sensitivity, and the like. In the methods, for measuring the lipid constituent, according to the invention, as shown in specific examples as described below, the measurement of the lipid constituent can be carried out by using one or more species of enzymes selected from enzymes catalyzing decomposition reaction, dehydrogenation reaction, and oxidation reaction. The lipid constituent contained in urine is, in comparison with the lipid constituent contained in serum, present in a very small amount and can be measured by using the enzyme with high sensitivity.

Specifically, a neutral fat is, for example, measured by decomposing the neutral fat into a glycerol and a fatty acid with lipase followed by measuring the produced glycerol by the enzyme reaction. The measurement of glycerol by the enzyme reaction includes the method by acting glycerol kinase and glycerol-3-phosphate dehydrogenase to measure a change rate from a coenzyme NAD to NADH, the method by acting glycerol kinase and glycerol phosphate oxidase to measure produced hydrogen peroxide, a high sensitivity method by acting glycerol kinase, glycerol-3-phosphate dehydrogenase, and glycerol phosphate oxidase to measure glycerol by cycling method, and the method by acting glycerol dehydrogenase.

For measurement of phospholipid, the method by acting phospholipase and glycerophosphoryl choline diesterase to produce glycerol-3-phosphoric acid for measuring glycerol-3-phosphoric acid in the same way as that of the neutral fat and other publicly known methods for measuring enzymatically phospholipids are used.

For measurement of cholesterols being a representative sterol in a living body, the following methods have been known: the method by acting cholesterol oxidization enzyme to produce hydrogen peroxide for measuring produced hydrogen peroxide by using peroxidase; the method by acting cholesterol dehydrogenase for measuring the change rate or a change amount from the coenzyme NAD to NADH, and also, the method for measuring cholesterol in high sensitivity by balanced amplifying reaction using two species of coenzymes, the coenzyme NADH and thioNAD, by using cholesterol dehydrogenase (JP P1996-70894 A); and the method for measurement by initiating the cycling reaction using cholesterol as a substrate in the presence of a reduction type substrate for a second dehydrogenase reproducing an oxidative coenzyme, which is converted by cholesterol oxidase or the like, to a reductive type coenzyme (JP P1999-18797 A).

In the case of fatty acid esters of cholesterol, working such decomposition enzyme as cholesterol esterase makes measurement by the above described methods possible after conversion to free cholesterol.

In the case of fatty acid, usable method includes a measurement of hydrogen peroxide produced by acting acyl coenzyme A synthetase and acyl coenzyme A oxidase.

For prostaglandin, measurement may be performed by acting a prostaglandin oxidase.

In the case of glycolipid, measurement can be performed by acting glycolipid decomposition lipase and other lipases to produce glycerol.

In the case of sphingolipid, it may be measured by acting sphingolipid decomposition lipase.

The method for measuring lipid constituent is not restricted to the methods exemplified above, but may be modified and/or added properly in accordance with the known measuring method for lipid constituents.

The invention provides the reagent, which is necessary for the method for measuring the lipoprotein or analogs thereof contained in urine or the constituent contained therein according to the invention and which is made as a kit or a single product. The reagent according to the invention may contain the surfactant for homogenization of the insoluble fat contained in urine by solubilizing or dispersing and reagents to quantify the lipid constituent. The reagent according to the invention may be a simple measuring device prepared by making a carrier contain a reagent necessary for the measurement.

### (Means for examination of nephropathy)

Another means for solving the problem according to the invention is means for examination of nephropathy, characterized in that measurement is carried out in proper combination of lipoprotein or analogs thereof contained in urine and constituents contained therein and the lipid constituent. So far, the examination of the urine neutral fat drop for detection of nephropathy was conducted by a complicated means such as microscopic observations. In the invention, on the basis of the findings in that a result of measurement of lipoprotein or analogs thereof contained in urine and constituents contained therein and the lipid constituent obtained by using the simple method, for measuring urine lipid constituents, according to the invention suggests a significant relation to nephropathy, the simple means for examination of nephropathy was achieved. In addition, a degree of nephropathy of a subject may be determined by scoring the amount of the urine substances described above.

For means for examination of nephropathy according to the invention, the method for measuring the lipoprotein or analogs thereof contained in urine or the constituent contained therein or the lipid constituent according to the invention may be applied and these constituents may be properly combined. The lipid constituent, which is contained in urine, as the object of the measurement is contained mainly in the fat droplet, the fat body, and/or the fat drop, and HDL-like lipoprotein in urine. And, objects of the measurement are one or more species of lipid constituents selected from, at least, the neutral fat and its derivative, and the phospholipid and the derivative thereof, the lipid peroxide, sterols, fatty acids, fatty acid salts, fatty acid esters, aliphatic alcohols, aliphatic aldehydes, lipoids, sphingolipids, prostaglandins, and carotinoids. As constituents contained in the lipoprotein or analogs thereof, in addition to lipids as described above, the protein readable with anti-human apoA-I antibody, the protein reactable with anti-human apoA-II antibody, and other apoproteins are exemplified to use as the object of the measurement. Moreover, the lipoprotein or analogs thereof is sufficiently a complex containing the lipid and the protein and exemplified by the chylomicron, VLDL, IDL, HDL, and analogs thereof.

In experimental examples, a cholesterol value and a phospholipid value of nephropathy patients were measured. These values were significantly higher in comparison with a healthy person. From this result, it was made clear that quantifying the lipid constituents at least cholesterol and/or phospholipid, which are contained in urine, is useful for finding of nephropathy. In addition, apoA-I and various urine constituents were measured before and after treatment of the patient having renal diseases. As the result, decrease in apoA-I was observed after treatment and its usefulness for diagnosis of nephropathy is suggested.

As described above, the invention provides means for examination of nephropathy, in which lipoprotein or analogs thereof contained in urine, constituents contained therein, and lipid constituents are measured and the result is analyzed to make detection of nephropathy and determination of the degree thereof possible.

The invention discloses that for detection of nephropathy and determination of the degree thereof, in addition to measurement of substances as described above, methods for measurement or detection of the surface antigen of the leukocyte contained in urine are applied in combination. Particularly, only for the sample showing a high value of substances as described above (for example, scoring the result of the measurement of lipids, lipoproteins, and/or apoproteins), methods for measurement or detection of the surface antigen of the leukocyte contained in urine may be applied. By these combinations, more preferable examination of nephropathy becomes possible. In other words, by combining of measurement of the urine lipid constituent with measurement or detection of the surface antigen of the leukocyte contained in urine, detection of nephropathy and determination of the degree thereof are performed more preferably.

In the case where the examination of nephropathy is performed in combination of measurement of substances, as described above, contained in urine with measurement or detection of the surface antigen of the leukocyte contained in urine, it is possible that a part of urine is used for measurement of substances as described above and also a part thereof was centrifuged to fractionate the urinary sediment fraction, followed by measuring and detecting the surface antigen such as CD14, CD16, and 25F9 of the leukocyte by publicly know microscopic observation or flow cytometry. In addition, it is possible that the above describe substances are measured by using a part of the urinary sediment fraction and the rest part is used for measurement or detection of the surface antigen of the leukocyte by microscopic observation or flow cytometry or the like. Moreover, a part of urine is used for fractionation of the urinary sediment by centrifugation and the surface antigen of the leukocyte is measured or detected. Moreover, it is possible that a part of urine is subjected to centrifugation to fractionate the urinary sediment followed by measuring and detecting the surface antigen of the leukocyte, and that another part of it is subjected to freezing and melting and/or treatment with the surfactant independently or in combination to measure by using a urine sample passed through a process for breaking cells and particular substances, which are contained in urine.

Analyses, which is included in the nephropathy-examining means according to the invention, of the results obtained by measurement of urine lipids, measurement of urine lipoproteins and/or urine apoproteins, or combination, if necessary, of these results with measurement or detection of the surface antigen of the leukocyte contained in urine allows more preferable determination of nephropathy. The results of measurements are properly chosen for use in the analysis. For example, a ratio of cholesterol to phospholipid may be adopted as an index and the ratio may be combined with the result of the measurement of CD14. Also for the result of the measurement and detection of the surface antigen of the leukocyte contained in urine, a plurality of results can be combined.

### Examples

The invention will be further described with reference examples below; however, the invention is not restricted to the examples.

### Example 1

A cholesterol concentration was measured for urine of healthy subjects and urine of nephropathy patients. Urine was previously blended with a 10% TritonX-100 in a proportion of 9: 1 to make a urine examination sample.

For cholesterol measurement, two kinds of reagents with the following composition were prepared for use for measurement and a 10 µL of the urine examination sample was taken, added to a 250 µL of reagent-1, and subjected to a reaction at a 37 deg. C for 5 min, and then, 100 µL of reagent-2 was added and a change of absorbance at a wavelength of 415 nm for every 1 min in the 1 min to 3 min after the addition was measured to calculate the concentration of cholesterol.

The result will be shown in Table 1.

| Reagent-1 | |
|---|---|
| PIPES | 20mmol/L |
| KCl | 150mmol/L |
| TritonX-100 | 0.3 W/V% |
| Sodium cholate | 0.4 W/V% |
| ThioNAD | 1.25mmol/L |
| Bovine serum albumin | 0.1 W/V% |
| Cholesterol dehydrogenase | 3U/ml |
| Cholesterol esterase | 5U/ml |
| pH | 7.0 |

| Reagent-2 | |
|---|---|
| CHES | 175mmol/L |
| TritonX-100. | 0.3 W/V% |
| Sodium cholate | 0.3 W/V% |
| β-NADH | 3.5mmol/L |
| pH | 9.1 |

### Example 2.

For the urine sample prepared in Example 1, a phospholipid concentration was measured. For measurement of phospholipid, two kinds of reagents with the following composition were prepared for use for measurement and the 10 µL of the urine examination sample was taken, added to the 250µL of reagent-1, and subjected to a reaction at a 37 deg. C for 5 min, and then, 100 µL of reagent-2 was added and the change of absorbance at a wavelength of 546 nm for every 1 min in the 1 min to 3 mm after the addition was measured to calculate the concentration of phospholipid.

The result will be shown in Table 1.

| Reagent-1 | |
|---|---|
| HEPES | 500mmol/L |
| CaCl₂ | 1.0mmol/L |
| TritonX-100 | 0.2 W/V% |
| Glycerol-3-phosphate dehydrogenase | 1.5U/ml |
| Phospholipase A1 | 2.0U/ml |
| Phospholipase A2 | 2.0U/ml |
| glycerophosphoryl choline phosphodiesterase | 0.6U/ml |
| NADH | 0.5mM |
| Phenol | 0.05% |
| Ascorbic acid oxidase | 3U/L |
| pH | 8.0 |

| Reagent-2 | |
|---|---|
| Phosphate buffer solution | 20mmol/L |
| glycerol-3-phosphate oxidase | 200U/ml |
| POD | 10U/ml |
| 4-aminoantipyrine | 2.0mM |
| pH | 7.5 |

As the result, it was found that concentrations of cholesterol and phospholipid, which were contained in urine of nephropathy patients, were distinctly higher than those of healthy subjects. Hence, it was suggested that measuring at least cholesterol and/or phospholipid, which were contained in urine, makes the examination of nephropathy possible.

### Table 1. Measurements of cholesterol and phospholipid, which were contained in urine of healthy subjects and nephropathy patients

①Urine sample
②Urine of healthy subject
③Urine of nephropathy patient
④Cholesterol
⑤Phospholipid

### Example 3

Concentrations of phospholipid, free cholesterol, cholesterol, and apoA-1 contained in urine samples of 122 cases (51 cases of IgA nephropathy [IgAN], 10 cases of diabetic nephropathy [DMN], 9 cases of nephrosclerosis, 6 cases of membranous nephropathy [MN], 7 cases of membranoproliferative glomerulonephritis [MPGN], 12 cases of rapidly progressive nephropathy [RPGN], 3 cases of minimal change nephrosis syndrome [MCNS], 12 cases of focal glomerulosclerosis [FGS], 5 cases of chronic renal failure [CRF], and 7 cases of Normal) were measured.

The results have been presented in Figs 1 to 5. It was found that any one of lipid-related markers examined in any disease groups of nephropathy as described above showed significant high values in comparison with normal urine. It is also found that measurement of lipid constituents contained in urine makes examination and diagnosis of nephropathy possible. Further, number (in a 5 mL) of an oil red O-positive particle (urine fat drop) reported by Oda et al. was counted (Oda, et al., Kidney International 53: 1190 - 1200, 1998).

The urine fat drop showed a particularly high value in IgAN, RPGN, and FGS and on the contrary, same values in MCNS and others. In contrast, it was found that the method in accordance with the invention can detect also other nephropathy variables with a high sensitivity in addition to IgAN, RPGN, and FGS and, therefore, usefulness of the invention was proven.

Meanwhile, measurement of cholesterol was conducted by applying the method shown in Example 1, measurement of phospholipid was conducted by applying the method shown in Example 2, and measurement of free cholesterol was conducted by applying the method same as that of Example 1 except for removing cholesterol esterase from reagent-1 presented in Example 1.

Measurement of apoA-1 was carried out by using ApoA-1 Auto-N "the first."

Urine was previously blended with 10% Triton-100 in the ratio of 9: 1 to be used as the urine sample. Operation was conducted after a handling manual, other than measurement in the reagent-sample ratio of 10 µL of urine sample: 160 µL of the first reagent: 40µL of the second reagent.

### Example 4.

By using 175 urine samples collected from subjects of periodic health examination, urine cholesterol was measured by the method described in Example 1 to yield a reference value of urine cholesterol of the healthy subject. A cholesterol value of serum of the healthy subject ranged normally 130 to 250 mg/dL and, on the contrary, the value of urine cholesterol ranged from 0.01 to 25 mg/dL, which is 1/1000 or smaller of serum cholesterol value.

As a control group, a commercial serum cholesterol measurement kit was used for measurement. However, cholesterol was never measured in any samples. From these results, it was known that measurement of urine cholesterol requires a high sensitivity measurement method by using the methods described in Example 1 according to the invention.

### Example 5.

Cholesterol contained in urine of the healthy subject and nephropathy patients was analyzed by HPLC and electrophoresis.

### (Preparation of sample)

Urine was concentrated by using ultrafiltration. Normal urine and patient urine were concentrated into about 130 times and about 10 times, respectively.

### (Experimental example 1: analysis by HPLC)

For a column, Superose 6 (diameter 1.0 cm X 30 cm) and Superose 12 (diameter 1.0 cm X 30 cm) (Pharmacia made) were used in a series. A buffer solution used was 10 mM Tris-HCl buffer, pH 7.5 (0.14MNaCl, 1.0 mM EDTA - 2Na, and 0.1% NaN₃ were contained) and the solution was sent in a flow rate of 0.3 mL/min. 100 µL of the sample prepared in the above described way was injected and 30 µL of serum was also injected as a standard sample. Fractionation was performed in every 2 min to measure the cholesterol concentration of individual fractions. The method shown in Example 1 was employed for measurement.

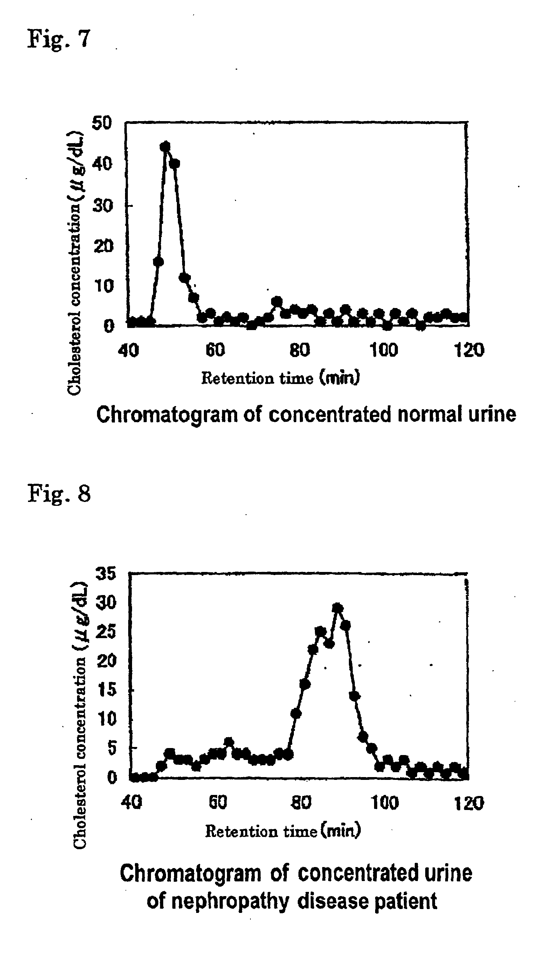

The result is shown in Figs 6 to 8. As shown in Figs 6 and 8, urine of the nephropathy patient showed a peak in apposition of the same retention time as that of the fraction of serum HDL. Therefore, it was suggested that a lipid fraction having the same molecular size as that of HDL is contained. As shown in Figs 6 and 7, normal urine showed the peak in the position of the molecular size larger than that of a serum VLDL fraction and thus, removal from the cell might be caused.

### (Experimental Example 2: analysis by electrophoresis)

Choletricombo of Helena Kenkyuusyo was employed as the system to operate electrophoresis using an agarose gel.

The normal and patient urine did not move from an original point of electrophoresis. This means no surface electric charge probably caused by modification of a surface protein.

A basal membrane of a glomerulus prevents a leak of blood protein or the like by an electric charge barrier. However, in consideration that HDL lost electric charges by modification caused by a disease, passing through the basal membrane can take place. Many of such modification is caused mainly by oxidation and therefore, it is possible that an oxidized lipoprotein expresses cytotoxicity by adhering to a kidney tubular epithelial cell.

### Example 6.

### (Comparison of urine constituents before and after therapeutic treatment)

Measurement was carried out for a very small amount of cholesterol (mCHO) in urine and constituents in urine or serum creatinine before and after therapeutic treatment for patients having renal diseases (RPGN). Constituents in urine were subjected to creatinine correction.

Cholesterol was measured by employing the method shown in Example 1, apoA-1 was measured by using ApoA-1 Auto-N "the first", albumin was measured by using m-ALB immune reagent (INTERNATIONAL REAGENTS CORP. made), protein was measured by using m-TP reagent (INTERNATIONAL REAGENTS CORP. made), N-acetyl β-D-glunosaminadase (NAG) was measured by using N-assay NAG "Nittobo" (Nittobo Corp. made), creatinine was measured by using CRE reagent LB "Kokusai" (INTERNATIONAL REAGENTS CORP. made).

Urine was previously blended with 20% Triton-100 in the ratio of 50: 1

The result will be presented in Figs. 9 to 13. ApoA-1 was not detected after therapeutic treatment. Other nephropathy disease marker, creatinine, NAG, TP, and ALB, were detected even after therapeutic treatment. Thus, ApoA-1 may be used for the examination of nephropathy in a different aspect from other markers.

### Industrial Applicability

As described above, the examination means according to the invention allows examining nephropathy more certainly.

## Claims

1. Method for examining nephropathy, **characterized by** measuring a lipoprotein or an analog thereof contained in urine, or a constituent contained the lipoprotein or the analog.

2. The method for examining nephropathy according to claim 1, characterize in that the lipoprotein or the analog thereof is a substance containing at least one protein selected from the group consisting of a protein reactable with an anti-human apoA-I antibody and a protein reactable with an anti-human apoA-II antibody.

3. The method for examining nephropathy according to claim 1 or 2, **characterized in that** the lipoprotein or the analog thereof is a high density lipoprotein or an analog thereof.

4. The method for examining nephropathy according to any of claims 1 to 3, **characterized by** measuring an apoprotein as the constituent contained in the lipoprotein or the analog thereof.

5. The method for examining nephropathy according to any of claims 1 to 4, **characterized by** measuring at least one protein selected from the group consisting of a protein readable with an anti-human apoA-I antibody and a protein reactable with an anti-human apoA-II antibody as the constituent contained in the lipoprotein or the analog thereof.

6. The method for examining nephropathy according to any of claims 1 to 5, **characterized by** further measuring a surface antigen of a urine leukocyte.

7. The method for examining nephropathy according to any of claims 1 to 6, **characterized by** further measuring a lipid constituent.

8. The method for examining nephropathy according to claim 7, **characterized in that** the lipid constituent is cholesterol.

9. A reagent for an examination, comprising a kit or a single product of a reagent necessary for the examination according to any of claims 1 to 8.
